# EUROPEAN PATENT APPLICATION

(11) **EP 2 564 849 A1**
(43) Date of publication of application: **06.03.2013**
(21) Application number: 11775089.3
(22) Date of filing: 27.04.2011
(51) Int. Cl.: A61K 31/4545, A61K 31/496, A61P 13/00, A61P 13/08, A61P 13/10, A61P 19/00, A61P 25/04, A61P 29/00, A61P 43/00, C07D 213/65, C07D 213/80, C07D 401/12, C07D 401/14, C07D 413/14

(54) **PROPHYLACTIC OR THERAPEUTIC AGENT FOR DISEASES ASSOCIATED WITH PAINS IN URINARY ORGANS**

(30) Priority: 28.04.2010 JP 2010104262
(71) Applicant: Astellas Pharma Inc., Tokyo 103-8411 (JP)
(72) Inventor: SOMEYA, Akiyoshi, Tokyo 103-8411 (JP); HAYASHIDA, Hiroko, Tokyo 103-8411 (JP); KODA, Mai, Tokyo 103-8411 (JP); TANAHASHI, Masayuki, Tokyo 103-8411 (JP); YOSHIOKA, Katsuro, Tokyo 103-8411 (JP)
(74) Representative: HOFFMANN EITLE
(86) International application number: PCT/JP2011/060332
(87) International publication number: WO 2011/136308

(57) **Abstract**

Disclosed is a prophylactic or therapeutic agent for diseases associated with pains in urinary organs. It is confirmed that a pyridyl non-aromatic nitrogenated heterocyclic-1-carboxylate compound or a salt thereof has an effective bladder capacity increasing activity and also has an analgesic activity on pains in the bladder and the testis, which rely on a FAAH inhibiting activity thereof. Therefore, a pyridyl non-aromatic nitrogenated heterocyclic-1-carboxylate compound or a salt thereof can be used for the prevention or treatment of interstitial cystitis/bladder pain syndrome and/or chronic non-bacterial prostatitis/chronic pelvic pain syndrome.

## Description

### Technical Field

The present invention relates to an agent for preventing or treating diseases accompanied by urinary pain, such as interstitial cystitis/bladder pain syndrome and chronic nonbacterial prostatitis/chronic pelvic pain syndrome.

### Background Art

Both of interstitial cystitis/bladder pain syndrome and chronic nonbacterial prostatitis/chronic pelvic pain syndrome are diseases that exhibit bladder pain as one of cardinal symptoms together with urinary frequency (Neurourology and Urodynamics, Vol. 21, pp 167-178, 2002; The Journal of Urology, Vol. 168, pp 593-598, 2002).
Interstitial cystitis is noninfectious cystitis that is frequently seen in females in their 20's to 60's. The cardinal symptoms of this disease include suprapubic pain, urinary frequency, and urinary urgency. However, there are no common diagnostic criteria or definite therapy for this disease so far. In 1987, the National Institute of Diabetic, Digestive and Kidney Disease (NIDDK) proposed strict entry criteria for research, and the criteria are currently taken as clinical diagnostic criteria and used for diagnosis in many cases. However, there is also criticism that the criteria are too strict to be applied. Therefore, an attempt at ascertaining the disease from symptoms by changing the name of the disease to "painful bladder syndrome" or "bladder pain syndrome" was made. In 2002, the International Continence Society proposed for the first time the definition of disease for painful bladder syndrome, which was "The complaint of suprapubic pain related to bladder filling, accompanied by other symptoms such as increased daytime and night-time frequency, in the absence of proven urinary infection or other obvious pathology". Currently, there is the definition of disease for interstitial cystitis/bladder pain syndrome, which is "An unpleasant sensation (pain, pressure, discomfort) perceived to be related to the urinary bladder, associated with lower urinary tract symptoms of more than six weeks duration, in the absence of infection of other identifiable causes" proposed in 2008 by the Society for Urodynamics and Female Urology of the USA.

Chronic nonbacterial prostatitis/chronic pelvic pain syndrome is one of typical urinary pain diseases, and is categorized as Category III among four categories of prostatitis syndromes proposed by the National Institute of Health (NIH) of the USA in 1999, as a group of diseases exhibiting pain/discomfort in the pelvic region including the perineum and the portion of the testes and the penis and symptoms relating to urination such as a feeling of residual urine and urinary frequency. However, unlike acute bacterial prostatitis in Category I or chronic bacterial prostatitis in Category II, the above disease is cryptogenic, so a definite therapy has not been found. Compared to other diseases exhibiting lower urinary tract symptoms such as benign prostatic hyperplasia, interstitial cystitis, or overactive bladder, chronic nonbacterial prostatitis/chronic pelvic pain syndrome exhibits pain in male reproductive organs including the testes as a characteristic symptom, so this characteristic is exemplified in NIH-Chronic Prostatitis Symptom Index (NIH-CPSI) as well.
As described above, a definite therapy for diseases accompanied by urinary pain, such as interstitial cystitis/bladder pain syndrome and chronic nonbacterial prostatitis/chronic pelvic pain syndrome has not been found, so it is urgently desired to develop a therapeutic agent that is excellently effective and safe.

Fatty acid amide hydrolase (FAAH) is known to hydrolyze endocannabinoids and cause endocannabinoids to lose activity (Prostaglandins Leukotrienes and Essential Fatty Acids, Vol. 66, pp 143-160, 2002; British Journal of Pharmacology, Vol. 141, pp 253-262, 2004; Nature, Vol. 384, pp 83-87, 1996; Biochemical Pharmacology, Vol. 62, pp 517-526, 2001). Endocannabinoid is a generic name for substances in the body that act on a cannabinoid receptor to exert physiological action. Typical examples of the endocannabinoids include anandamide, palmitoylethanolamide, oleamide, and 2-arachidonoyl glycerol, and these are known to lose their activity by being hydrolyzed by FAAH. In addition, Δ9-tetrahydrocannabinol considered to be an active ingredient of marihuana is known to activate the cannabinoid receptor (Current Medicinal Chemistry, Vol. 6, pp 635-664, 1999).

So far, two types of cannabinoid receptors including CB 1 and CB2 are known in mammals. CB 1 is expressed in the central and peripheral nervous systems, and its activation induces a mental process, an analgesic action, and the like. CB2 is expressed in the tissue of the immune system, and its activation induces an anti inflammatory action, an analgesic (inflammatory) action, and the like.

Meanwhile, in a rat cystitis model, cannabinoid receptor agonist increases bladder capacity and urination threshold (The Journal of Neuroscience, Vol. 22, pp 7147-7153, 2002; Pain, Vol. 76, pp 189-199, 1998), and adverse effects such as hallucination, delusion, tachycardia, and orthostatic hypotension that are observed when the cannabinoid receptor agonist is administered to an animal are not observed when FAAH inhibitor is administered (Nature Medicine, Vol. 9, pp 76-81, 2003). From these facts, FAAH inhibitor is expected to be an agent for treating interstitial cystitis/bladder pain syndrome and/or chronic nonbacterial prostatitis/chronic pelvic pain syndrome that causes less concern of adverse effects or habitual use.

A large number of compounds having a FAAH inhibitory activity have been reported, and for example, the following compounds are known.

Patent Document 1 discloses a pyridyl non-aromatic nitrogen-containing heterocyclic-1-carboxylate compound as a compound useful for treating urinary frequency, urinary incontinence, overactive bladder, pain, and the like. However, Patent Document 1 does not specifically disclose the effectiveness with respect to treatment of interstitial cystitis/bladder pain syndrome and/or chronic nonbacterial prostatitis/chronic pelvic pain syndrome.

Patent Document 2 discloses a pyridyl non-aromatic nitrogen-containing heterocyclic-1-carboxylate compound as a compound useful for treating neuropathic pain. However, Patent Document 2 does not specifically disclose the effectiveness with respect to treatment of interstitial cystitis/bladder pain syndrome and/or chronic nonbacterial prostatitis/chronic pelvic pain syndrome.

Patent Document 3 discloses that aryl and heteroaryl piperidine carboxylic acid derivatives are useful for treating urinary incontinence or cystitis as a form of disease including a large number of listed diseases. However, Patent Document 3 does not disclose data that specifically show the effectiveness with respect to treatment of urinary incontinence or cystitis.

Patent Document 4 discloses that an amide compound is useful for treating interstitial cystitis as a form of disease including a large number of listed diseases. However, Patent Document 4 does not disclose data that specifically show the effectiveness with respect to treatment of interstitial cystitis.

[Patent Document 1] Pamphlet of International Publication WO2006/088075
[Patent Document 2] Pamphlet of International Publication WO2010/007966
[Patent Document 3] Pamphlet of International Publication WO2005/090347
[Patent Document 4] Pamphlet of International Publication WO2006/054652

### Disclosure of Invention

### Problems to Be Solved by the Invention

An object of the present invention is to provide an agent for preventing or treating diseases accompanied by urinary pain, such as interstitial cystitis/bladder pain syndrome and chronic nonbacterial prostatitis/chronic pelvic pain syndrome.

### Means for Solving the Problems

The present inventors thoroughly examined compounds having FAAH inhibitory activity. As a result, they found that a pyridyl non-aromatic nitrogen-containing heterocyclic-1-carboxylate compound of Formula (I) (hereinafter, described as a "compound of Formula (I)" in some cases) or a salt thereof has not only an action of increasing effective bladder capacity but also an analgesic action against bladder pain and testis pain based on FAAH inhibitory action, and is useful for preventing or treating interstitial cystitis/bladder pain syndrome and/or chronic nonbacterial prostatitis/chronic pelvic pain syndrome, thereby completing the present invention.
That is, the present invention relates to an agent for preventing or treating interstitial cystitis/bladder pain syndrome and/or chronic nonbacterial prostatitis/chronic pelvic pain syndrome, that is, a pharmaceutical composition for preventing or treating interstitial cystitis/bladder pain syndrome and/or chronic nonbacterial prostatitis/chronic pelvic pain syndrome, comprising a compound of Formula (I) or a salt thereof as an active ingredient. 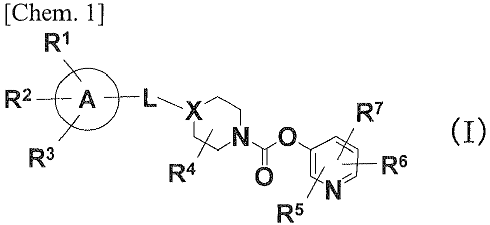 (wherein,
A represents benzene ring, 5- to 7-membered cycloalkane ring, or 5- to 7-membered nitrogen-containing hetero ring;
L represents single bond, lower alkylene, lower alkenylene, -N(R⁸)-CO-, -CO-N(R⁸)-, - lower alkenylene-CO-, -O-, or -CO-;
R⁸ represents H or lower alkyl;
X represents CH or N;
R¹, R², and R³ are the same as or different from each other and represent H, halogen, -CN, -CF₃, lower alkyl, -O-lower alkyl, aryl which may be substituted with group(s) selected from the following G group, nitrogen-containing heteroaryl which may be substituted with group(s) selected from the following G group, R⁹-lower alkylene-O-, R⁹-lower alkylene-N(R⁸)-, or R¹⁰R¹¹N-CO-;
R⁹ represents aryl which may be substituted with group(s) selected from the following G group, nitrogen-containing heteroaryl which may be substituted with group(s) selected from the following G group, or 5- to 7-membered cycloalkyl;
R¹⁰ and R¹¹ are the same as or different from each other and represent H or lower alkyl, or form 5- to 7-membered nitrogen-containing hetero ring together with N atom bonded thereto;
G group consists of halogen, -CN, -CF₃, lower alkyl, and -O-lower alkyl;
R⁴ represents H or lower alkyl; and
R⁵, R⁶, and R⁷ are the same as or different from each other and represent H, lower alkyl, - CO-O-lower alkyl, -CO₂H, or -CONH₂.)

Moreover, the present invention relates to a method of preventing or treating interstitial cystitis/bladder pain syndrome and/or chronic nonbacterial prostatitis/chronic pelvic pain syndrome, comprising administering an effective amount of the compound of Formula (I) or a salt thereof to a mammal. The present invention also relates to use of the compound of Formula (I) or a salt thereof for manufacturing an agent for preventing or treating interstitial cystitis/bladder pain syndrome and/or chronic nonbacterial prostatitis/chronic pelvic pain syndrome, use of the compound of Formula (I) or a salt thereof for preventing or treating interstitial cystitis/bladder pain syndrome and/or chronic nonbacterial prostatitis/chronic pelvic pain syndrome, and the compound of Formula (I) or a salt thereof for use in prevention or treatment of interstitial cystitis/bladder pain syndrome and/or chronic nonbacterial prostatitis/chronic pelvic pain syndrome.

### Effects of the Invention

The pyridyl non-aromatic nitrogen-containing heterocyclic-1-carboxylate compound of Formula (I) or a salt thereof which is an active ingredient of the present invention can be used as an agent for preventing or treating interstitial cystitis/bladder pain syndrome and/or chronic nonbacterial prostatitis/chronic pelvic pain syndrome.

### Embodiments for Carrying Out the Invention

Hereinafter, the present invention will be described in detail.
The compound of Formula (I) or a salt thereof that is an active ingredient of the agent for preventing or treating interstitial cystitis/bladder pain syndrome and/or chronic nonbacterial prostatitis/chronic pelvic pain syndrome of the present invention is the compound disclosed in Patent Documents 1 and 2, and can be prepared based on the disclosure of the Patent Documents.

In the present specification, "lower alkyl," is linear or branched alkyl having 1 to 6 carbon atom(s) (hereinafter, abbreviated to C₁₋₆), and examples thereof include methyl, ethyl, n-propyl, isopropyl, n-butyl, isobtutyl, sec-butyl, tert-butyl, n-pentyl, n-hexyl, and the like. Another embodiment thereof is C-₁₋₄ alkyl, and still another embodiment thereof is methyl or ethyl.

The "lower alkylene" is linear or branched C₁₋₆ alkylene, and examples thereof include methylene, ethylene, trimethylene, tetramethylene, pentamethylene, hexamethylene, propylene, methyl methylene, ethyl ethylene, 1,2-dimethylethylene, 1,1,2,2-tetramethylethylene, and the like. Another embodiment thereof is methylene, ethylene, trimethylene, or pentamethylene.

The "lower alkenylene" is linear or branched C₂₋₆ alkenylene, and examples thereof include vinylene, ethylidene, propenylene, butenylene, pentenylene, hexenylene, 1,3-butadienylene, 1,3-pentadienylene, and the like. Another embodiment thereof is C₂₋₄ alkenylene, and still another embodiment thereof is vinylene.

The "5- to 7-membered cycloalkyl" is C₅₋₇ saturated hydrocarbon ring group, which is specifically cyclopentyl, cyclohexyl, or cycloheptyl. Another embodiment thereof is cyclohexyl. The "5- to 7-membered cycloalkane ring refers to a ring constituting the "5- to 7-membered cycloalkyl", which is specifically cyclopentane ring, cyclohexane ring, or cycloheptane ring.

The "halogen" refers to F, Cl, Br, or I.

The "aryl" is C₆₋₁₄ monocyclic to tricyclic aromatic hydrocarbon ring group, and examples thereof include phenyl, naphthyl, and the like. Another embodiment thereof is phenyl.

The "5- to 7-membered nitrogen-containing hetero ring" refers to a 5- to 7-membered monocyclic saturated or unsaturated ring that contains 1 to 4 hetero atom(s) selected from O, S, and N and essentially contains at least 1 or more N atom(s). The unsaturated ring includes an aromatic hetero ring. In addition, S or O as a ring atom may be oxidized so as to form oxide or dioxide. Specific examples of the ring include pyrrolidine, imidazolidine, pyrazolidine, oxazolidine, thiazolidine, piperidine, piperazine, morpholine, thiomorpholine, azepane, diazepane, pyrroline, dihydropyridine, tetrahydropyridine, azepine, pyrrole, imidazole, pyrazole, triazole, tetrazole, oxazole, isoxazole, thiazole, isothiazole, oxadiazole, thiadiazole, pyridine, pyrimidine, pyridazine, pyrazine, triazine, and the like.

The "nitrogen-containing heteroaryl" refers to 5- to 10-membered monocyclic or bicyclic aromatic ring group that contains 1 to 4 hetero atom(s) selected from O, S, and N, and essentially contains at least one or more N atom(s). Specific examples thereof include pyrrolyl, imidazolyl, pyrazolyl, triazolyl, tetrazolyl, oxazolyl, isoxazolyl, thiazolyl, isothiazolyl, oxadiazolyl, thiadiazolyl, pyridyl, pyrimidinyl, pyrazinyl, pyridazinyl, triazinyl, indolyl, isoindolyl, benzimidazolyl, indazolyl, quinolyl, isoquinolyl, quinazolinyl, quinoxalinyl, phthalazyl, benzothiazolyl, benzisothiazolyl, benzoxazolyl, benzisoxazolyl, and the like.

In the present specification, the term "may be substituted" means that a group may be unsubstituted or may have 1 to 5 substituent(s). When a group has a plurality of substituents, these substituents may be the same as or different from each other.

In the present specification, the "urinary pain" means pain/pressure/discomfort in the suprapubic region including the bladder and pain/discomfort or the like of the pelvic region including the perineum, the portion of the testes and the penis, and the like.

An embodiment of the compound of Formula (I) or a salt thereof that is an active ingredient of the agent for preventing or treating interstitial cystitis/bladder pain syndrome and/or chronic nonbacterial prostatitis/chronic pelvic pain syndrome of the present invention is a compound or a salt thereof selected from a group consisting of pyridin-3-yl 4-{4-[(3-fluorobenzyl)oxy]phenoxy}piperidine-1-carboxylate (hereinafter, described as "compound A"), 5-{[(4-{4-[(3-fluorobenzyl)oxy]phenoxy}piperidin-1-yl)carbonyl]oxy}nicotinic acid (hereinafter, described as "compound B"), 5-({[4-(2-phenylethyl)piperidin-1-yl]carbonyl}oxy)nicotinic acid (hereinafter, described as "compound C"), 5-[({4-[4-(2-cyclohexylethoxy)phenoxy]piperidin-1-yl}carbonyl)oxy]nicotinic acid (hereinafter, described as "compound D"), 5-[({4-[(E)-2-phenylvinyl]piperidin-1-yl}carbonyl)oxy]nicotinic acid (hereinafter, described as "compound E"), 5-{[(4-{3-[1-(6-methylpyridin-2-yl)piperidin-4-yl]propyl}piperidin-1-yl)carbonyl]oxy}nicotinic acid (hereinafter, described as "compound F"), 5-(aminocarbonyl)pyridin-3-yl4-{2-[3-(aminocarbonyl)phenyl]ethyl}piperidine-1-carboxylate (hereinafter, described as "compound G"), 5-(aminocarbonyl)pyridin-3-yl4-(2- {3-[(dimethylamino)carbonyl]phenyl}ethyl)piperidine-1-carboxylate (hereinafter, described as "compound H"), 5-(aminocarbonyl)pyridin-3-yl4-{2-[3-(piperidin-1-ylcarbonyl)phenyl]ethyl}piperidine-1-carboxylate (hereinafter, described as "compound I"), 5-(aminocarbonyl)pyridin-3-yl 4-{2-[3-(pyrrolidin-1-ylcarbonyl)phenyl]ethyl}piperidine-1-carboxylate (hereinafter, described as "compound J"), pyridin-3-yl 4-[(2E)-3-phenylprop-2-enoyl]piperazine-1-carboxylate (hereinafter, described as "compound K"), pyridin-3-yl 4-(anilinocarbonyl)piperidine-1-carboxylate (hereinafter, described as "compound L"), 5-(aminocarbonyl)pyridin-3-yl 4-(2-phenylethyl)piperidine-1-carboxylate (hereinafter, described as "compound M"), pyridin-3-yl 4-(2-phenylethyl)piperazine-1-carboxylate (hereinafter, described as "compound N"), 5-(methoxycarbonyl)pyridin-3-yl 4-(2-phenylethyl)piperazine-1-carboxylate (hereinafter, described as "compound O"), 5-(aminocarbonyl)pyridin-3-yl 4-[2-(3-fluorophenyl)ethyl]piperidine-1-carboxylate (hereinafter, described as "compound P"), 5-(aminocarbonyl)pyridin-3-yl 4-[2-(3-cyanophenyl)ethyl]piperidine-1-carboxylate (hereinafter, described as "compound Q"), 5-(aminocarbonyl)pyridin-3-yl 4-(5-phenylpentyl)piperazine-1-carboxylate (hereinafter, described as "compound R"), pyridin-3-yl 4-(3-phenyl-1H-1,2,4-triazol-5-yl)piperidine-1-carboxylate (hereinafter, described as "compound S"), 6-methylpyridin-3-yl 4-[3-(4-fluorophenyl)-1H-1,2,4-triazol-5-yl]piperidine-1-carboxylate (hereinafter, described as "compound T"), 6-methylpyridin-3-yl 4-[5-(4-fluorophenyl)-1,3-oxazol-2-yl]piperidine-1-carboxylate (hereinafter, described as "compound U"), 2,6-dimethylpyridin-3-yl 4-[5-(3,4-difluorophenyl)-1,2,4-oxadiazol-3-yl]piperidine-1-carboxylate (hereinafter, described as "compound V"), 2-methylpyridin-3-yl 4-[3-(2-fluorophenyl)-1H-1,2,4-triazol-5-yl]piperidine-1-carboxylate (hereinafter, described as "compound W"), and 6-methylpyridin-3-yl 4-(3-phenyl-1H-pyrazol-1-yl)piperidine-1-carboxylate (hereinafter, described as "compound X").
The compounds A to R are the compounds disclosed in Patent Document 1, and the compounds S to X are the compound disclosed in Patent Document 2.

The compound of Formula (I) has tautomers or geometric isomers depending on the types of substituents. In the present specification, the compound of Formula (I) is described only in the form of an isomer in some cases, but the present invention also includes other isomers, separated isomers, or a mixture thereof.
In addition, the compound of Formula (I) has asymmetric carbon atom(s) or axial asymmmetry in some cases, and there may be optical isomers based on this. The present invention also includes separated optical isomers of the compound of Formula (I) or a mixture thereof.

A salt of the compound of Formula (I) is a pharmaceutically acceptable salt of the compound of Formula (I), and forms an acid addition salt or a salt with a base in some cases depending on the types of substituents. Specific examples thereof include acid addition salts with inorganic acids such as hydrochloric acid, hydrobromic acid, hyroiodic acid, sulfuric acid, nitric acid, and phosphoric acid or with organic acids such as formic acid, acetic acid, propionic acid, oxalic acid, malonic acid, succinic acid, fumaric acid, maleic acid, lactic acid, malic acid, mandelic acid, tartaric acid, dibenzoyl tartaric acid, ditoluoyl tartaric acid, citric acid, methanesulfonic acid, ethanesulfonic acid, benzenesulfonic acid, p-toluenesulfonic acid, aspartic acid, or glutamic acid, salts with inorganic bases such as sodium, potassium, magnesium, calcium, and aluminum or with organic bases such as methylamine, ethylamine, ethanolamine, lysine, and ornithine, salts with various amino acids such as acetylleucine or with amino acid derivatives, ammonium salts, and the like.

The active ingredient of the present invention also includes various hydrates or solvates, and any of crystalline polymorphs of the compound of Formula (I) and a salt thereof. In addition, the present invention also includes compounds labeled with various radioisotopes or non-radioisotopes.

The pharmaceutical composition that contains one or two or more kinds of the compound of Formula (I) or a salt thereof as an active ingredient can be prepared by generally used methods by using excipients that are generally used in the field of related art, that is, by using excipients or carriers for medications.
The composition may be administered by oral administration using tablets, pills, capsules, granules, powders, liquids, etc., or by parenteral administration using an intraarticular, intravenous, or intramuscular injections, suppositories, eye drops, eye ointments, transdermal liquids, ointments, transdermal patches, transmucosal liquids, transmucosal patches, inhalation agents, and the like.

As the solid composition for oral administration, a tablet, powder, granules, and the like are used. For the solid composition, one or two or more kinds of active ingredients are mixed with at least one kind of inactive excipient, for example, lactose, mannitol, glucose, hydroxypropyl cellulose, microcrystalline cellulose, starch, polyvinyl pyrrolidone, and/or magnesium aluminometasilicate. According to common methods, the composition may contain inactive additives, for example, lubricants such as magnesium stearate, disintegrating agents such as sodium carboxymethyl starch, stabilizers, and solubilizing agents. The tablet or pill may optionally be coated with sugar or with a film of gastric or enteric material if necessary.
The liquid composition for oral administration includes a pharmaceutically acceptable emulsions, solutions, suspensions, syrups, elixirs, or the like, and contains a generally used inactive diluent, for example, purified water or ethanol. The liquid composition may contain auxiliary agents such as solubilizers, moisturizers, or suspensions, sweeteners, flavors, aromatics, and antiseptics, in addition to an inactive diluent.

The injection for parenteral administration contains sterile aqueous or non-aqueous solutions, suspensions, or emulsions. Examples of the aqueous solutions include distilled water for injection and physiological saline. Examples of the non-aqueous solutions include propylene glycol, polyethylene glycol, plant oils such as olive oil, alcohols such as ethanol, polysorbate 80 (pharmacopoeial name), and the like. These compositions may further contain tonicity agents, antiseptics, moisturizers, emulsifiers, dispersants, stabilizers, or solubilizers. These are sterilized by filtration through a bacteria retaining filter, blending of a germicide, or irradiation. Moreover, these can be used by being prepared as a sterile solid composition and dissolved or suspended in sterile water or a sterile solvent for injection before use.

Examples of agents for external use include ointments, plasters, creams, jellies, cataplasms, sprays, lotions, eye drops, eye ointments, and the like. The agent for external use contains generally ointment bases and lotion bases, aqueous or non-aqueous liquids, suspensions, emulsions, and the like. Examples of the ointment bases or lotion bases include polyethylene glycol, propylene glycol, white vaseline, bleached beeswax, polyoxyethylene hydrogenated castor oil, glyceryl monostearate, stearyl alcohol, cetyl alcohol, lauromacrogol, sorbitan sesquioleate, and the like.

Transmucosal agents such as inhalation agents and transnasal agents are used in the form of solid, liquid or a semisolid, and can be prepared according to conventional known methods. For example, known excipients, pH adjustors, antiseptics, surfactants, lubricants, stabilizers, thickeners or the like may be appropriately added thereto. For administration, appropriate devices for inhalation or insufflation can be used. For example, by using known devices such as a metered dose inhaler or an atomizer, the compound can be administered alone or administered as powder of a formulated mixture or as a solution or suspension which is a combination of the compound with a pharmaceutically acceptable carrier. A dry powder inhaler and the like may be for single administration or multiple administration, and dry powders or powder-containing capsules can be used. Alternatively, the compound may be administered in the form of a pressurized aerosol spray using an appropriate propellant, for example, suitable gas such as chlorofluoroalkane, hydrofluoroalkane, or carbon dioxide.

Generally, in the case of oral administration, an appropriate daily dose is about 0.001 mg/kg to 100 mg/kg in terms of body weight, preferably 0.1 mg/kg to 30 mg/kg, and more preferably 0.1 mg/kg to 10 mg/kg, which is administered once or two to four times in separate doses. In the case of intravenous administration, an appropriate daily dose is about 0.0001 mg/kg to 10 mg/kg in terms of body weight, which is administered once or plural times in separate doses. In addition, the transmucosal agent is administered once a day or plural times a day in separate doses, in a dose of about 0.001 mg/kg to 100 mg/kg in terms of body weight. The dose is appropriately determined case by case in consideration of the symptoms, age, sex, and the like.

The compound of Formula (I) can be used in combination with various agents for preventing or treating interstitial cystitis/bladder pain syndrome and/or chronic nonbacterial prostatitis/chronic pelvic pain syndrome. In combined administrations, medicaments may be administered simultaneously, administered sequentially one by one, or administered at a desired time interval. In simultaneous administration, a combined medicament may be formulated or medicaments separately formulated may be used.

### Examples

Hereinafter, the present invention will be described in detail based on examples, but the present invention is not limited to the scope described in the following examples.

### Example 1

### Screening of Substance Inhibiting FAAH Activity by Using Human Bladder Epithelial Carcinoma-Derived Cell Line

### (1) Screening of substance inhibiting FAAH activity

Human bladder epithelial carcinoma-derived cell line, 5637 cells (HTB-9; ATCC) were seeded in a 48-well cell culture plate in an amount of 1 x 10⁵ cells/well by using RPMI 1640 medium (Invitrogen) containing 10% fetal bovine serum (HyClone), followed by incubation at 37°C for 12 hours or longer, and then the cells in each well were washed with 400 µl of a buffer (Hank's Balanced Salt Solution, 20 mM Hepes-NaOH (pH 7.4)). A test substance dissolved in DMSO was added to a substrate solution (the above buffer containing 3 µCi/ml radiolabeled anandamide (anandamide [ethanolamine 1-³H]) and 10 µM anandamide) so as to have a concentration from 0.003 nM to 30 nM, and as a control, DMSO was added alone. 100 µl/well of the substrate solution was added to the cells, followed by incubation in a CO₂ incubator at 37°C for 30 minutes. Thereafter, the cell culture plate was transferred onto ice, the substrate solution was removed by suction, and 75 µl/well of a cytolytic solution that have been ice-cooled (the above buffer containing 0.5% TritonX-100 and 10 µM of a compound cyclohexylcarbamic acid 3'-carbamoylbiphenyl-3-yl ester(URB597; Cayman Chemical Company; Kathuria et al., Nature Med., Vol. 9, pp 76-81, 2003) having FAAH inhibitory activity) was added thereto, followed by stirring. The obtained cell lysate was transferred from each well to a sample tube having a volume of 1.5 ml, and 150 µl of a solution including chloroform and methanol at a ratio of 1:1 (volume ratio) was added thereto, followed by stirring. By centrifugation (15000 rpm for 2 minutes), the solution was divided into the upper layer (water/methanol layer) containing the decomposed product ethanolamine (ethanolamine 1-³H) and the lower layer (chloroform layer) containing unreacted radiolabeled anandamide. 25 µl of the upper layer was transferred to a 96-well organic solvent-resistant white microplate (PicoPlate-96; PerkinElmer Inc.), and 150 µl of MicroScint 20 (PerkinElmer Inc.) was added thereto, followed by measurement by using a microplate scintillation counter (TopCount^{™}; Beckman Coulter, Inc.). A substance decreasing a measured value compared to the control was selected as a substance inhibiting FAAH activity.

### (2) Measurement of IC₅₀ value of substance inhibiting FAAH activity

A test compound dissolved in DMSO at a concentration of 10 mM was added to the substrate solution so as to have a concentration of 0.003 nM to 30 nM, and the effect exerted on the FAAH activity was investigated by the method described above. DMSO as a negative control and URB597 as a positive control were added to the substrate solution so as to a concentration of 10 µM. The measurement value of the positive control was set to 0%, and the measurement value of the negative control was set to 100%, whereby the IC₅₀ value was calculated. The results are shown in Table 1.

**[Table 1]**

| Test compound | IC₅₀ (nM) | Test compound | IC₅₀ (nM) |
|---|---|---|---|
| Compound A | 0.11 | Compound M | 0.081 |
| Compound B | 0.44 | Compound N | 0.18 |
| Compound C | 0.89 | Compound O | 1.4 |
| Compound D | 0.22 | Compound P | 0.058 |
| Compound E | 1.5 | Compound Q | 0.062 |
| Compound F | 0.89 | Compound R | 0.083 |
| Compound G | 0.50 | Compound S | 0.35 |
| Compound H | 0.54 | Compound T | 1.8 |
| Compound I | 0.16 | Compound U | 0.56 |
| Compound J | 0.52 | Compound V | 2.6 |
| Compound K | 0.58 | Compound W | 0.38 |
| Compound L | 0.69 | Compound X | 0.11 |

### Example 2

### Action of Compound with Respect to Rat with Cyclophosphamide (CPA)-Induced Urinary Frequency

The action of the compound improving bladder irritation symptom was examined using a pathological model. Cyclophosphamide (CPA) is known to be converted into the metabolite acrolein when administered systemically, and damage bladder mucosa via urine. In a rat, the administration of CPA induces bladder pain or urinary frequency accompanied by hemorrhagic cystitis, so drug efficacy with respect to these symptoms can be evaluated. For the experiment, 9-week-old female Wistar rats (Charles River Laboratories Japan, Inc.) were used. Two days after CPA (100 mg/kg) was administered intraperitoneally, the experiment was performed. By using 0.5% methyl cellulose as a solvent, a test compound was orally administered, and after 15 minutes, distilled water (30 ml/kg) was orally administered forcedly. The rat was put in a metabolic cage, and the weight of urine voided was continuously measured for 1 hour. The total amount of urine voided was divided by the total frequency of voiding to calculate effective bladder capacity. As a result, in the solvent-administered group, the effective bladder capacity was reduced, and urinary frequency was confirmed. On the other hand, the effective oral administration dose was 3 mg/kg for compounds A and B and 1 mg/kg for compounds C, D, E, F, G, H, J, L and X, and these compounds increased the reduced effective bladder capacity and improved the urinary frequency.

### Example 3

### Action of Compound with Respect to Rat Model with Bladder Pain

The analgesic action of the compound against bladder pain was examined using a pathological model. Cyclophosphamide (150 mg/kg) was administered intraperitoneally, and after two days, physiological saline was injected under a non-restraint condition, at a rate of 45 ml/h via a cannula inserted transurethrally into the bladder, thereby rapidly expanding the bladder. Due to rapid expansion of the bladder, amplification of electromyogram spikes in external oblique abdominal muscle accompanying behavior relating to pain was confirmed. The injection amount at this point in time can be taken as a threshold of bladder pain, which makes it possible to evaluate drug efficacy with respect to the threshold of bladder pain. For the experiment, 7-week-old male SD rats (Charles River Laboratories Japan, Inc.) were used (n=3-6). For the rats pre-treated with cyclophosphamide, the threshold of bladder pain before and after the administration of a test compound was measured, and the change in amount (Δ ml) was investigated. In order to obtain a value before administration, bladder expansion was performed until a stabilized threshold of bladder pain was consecutively obtained three times, and the average of the threshold of bladder pain of the final three times was taken as a measured value. By using 0.5% methyl cellulose as a solvent, a test compound (3 mg/5 ml/kg) was orally administered, and then after 60 minutes or 120 minutes, the threshold of bladder pain started to be measured. The average of the threshold of bladder pain consecutively measured three times was taken as a value measured after administration. The results are shown in Table 2. In the compound-administered group, the threshold of bladder pain was significantly increased compared to the solvent-administered group (p<0.05 for all, test method: Student's t-test), and the analgesic action of the compound against bladder pain was confirmed.

**[Table 2]**

| Test compound | Administration of compound | Threshold of bladder pain (amount of physiological saline injected (Δ ml)) | |
|---|---|---|---|
| | | Solvent-administered group | Compound-administered group |
| Compound C | 60 minutes before measurement | 0.011 | 0.145 |
| Compound J | 60 minutes before measurement | 0.011 | 0.150 |
| Compound L | 120 minutes before measurement | -0.003 | 0.173 |
| Compound X | 120 minutes before measurement | 0.017 | 0.138 |

### Example 4

### Action of Compound with Respect to Rat Model with Testis Pain

The analgesic action of the compound against testis pain was examined using a pathological model. If 1% acetic acid is administered at 1 ml/kg to right and left testes of a rat by using an injection needle, pain behavior (writhing) accompanied by testis pain is observed, which makes it possible to evaluate drug efficacy on the pain behavior. For the experiment, 3- to 4-week-old male Wistar rats (Charles River Laboratories Japan, Inc.) were used (n=9-13). By using 0.5% methyl cellulose as a solvent, a test compound (3 mg/5 ml/kg) was orally administered, and then after 55 minutes or 115 minutes, acetic acid was administered into the testis. The rats were transferred to a cylindrical acryl cage having a diameter of 30 cm and a height of 50 cm, and the number of times of pain behavior shown for 5 minutes to 15 minutes after the acetic acid administration was measured. The results are shown in Table 3. In the compound-administered group, the number of times of pain behavior was significantly reduced compared to the solvent-administered group (p<0.01 for all, test method: Student's t-test), and the analgesic action of the compound against testis pain was confirmed.

**[Table 3]**

| Test compound | Administration of compound | Number of times of pain behavior | |
|---|---|---|---|
| | | Solvent-administered group | Compound-administered group |
| Compound C | 60 minutes before measurement | 23.9 | 10.4 |
| Compound J | 60 minutes before measurement | 22.7 | 9.5 |
| Compound L | 120 minutes before measurement | 22.0 | 6.7 |
| Compound X | 120 minutes before measurement | 25.6 | 13.3 |

From the results of the aforestated respective experiments, it was confirmed that these compounds had not only the action of increasing effective bladder capacity, but also the analgesic action against bladder pain and testis pain, based on FAAH inhibitory action. Accordingly, the compound of Formula (I) or a salt thereof can be used for preventing or treating interstitial cystitis/bladder pain syndrome and/or chronic nonbacterial prostatitis/chronic pelvic pain syndrome.

### Industrial Applicability

The pyridyl non-aromatic nitrogen-containing heterocyclic-1-carboxylate compound of Formula (I) or a salt thereof as an active ingredient of the present invention can be used as an agent for preventing or treating interstitial cystitis/bladder pain syndrome and/or chronic nonbacterial prostatitis/chronic pelvic pain syndrome.

## Claims

1. An agent for prevention or treatment of interstitial cystitis/bladder pain syndrome and/or chronic nonbacterial prostatitis/chronic pelvic pain syndrome, comprising a compound of Formula (I) or a salt thereof as an active ingredient, 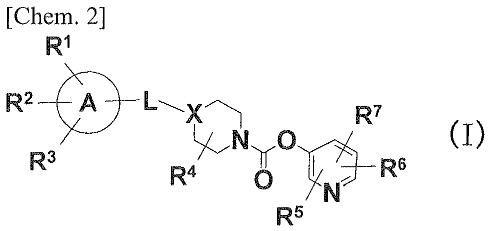 (wherein,
A represents benzene ring, 5- to 7-membered cycloalkane ring, or 5- to 7-membered nitrogen-containing hetero ring;
L represents single bond, lower alkylene, lower alkenylene, -N(R⁸)-CO-, -CON(R⁸)-, -lower alkenylene-CO-, -O-, or -CO-;
R⁸ represents H or lower alkyl;
X represents CH or N;
R¹, R², and R³ are the same as or different from each other and represent H, halogen, -CN, -CF₃, lower alkyl, -O-lower alkyl, aryl which may be substituted with group(s) selected from the following G group, nitrogen-containing heteroaryl which may be substituted with group(s) selected from the following G group, R⁹-lower alkylene-O-, R⁹-lower alkylene-N(R⁸)-, or R¹⁰R¹¹N-CO-;
R⁹ represents aryl which may be substituted with group(s) selected from the following G group, nitrogen-containing heteroaryl which may be substituted with group(s) selected from the following G group, or 5- to 7-membered cycloalkyl;
R¹⁰ and R¹¹ are the same as or different from each other and represent H or lower alkyl, or form 5- to 7-membered nitrogen-containing hetero ring together with N atom bonded thereto;
G group consists of halogen, -CN, -CF₃, lower alkyl, and -O-lower alkyl;
R⁴ represents H or lower alkyl; and
R⁵, R⁶, and R⁷ are the same as or different from each other and represent H, lower alkyl, -CO-O-lower alkyl, -CO₂H, or -CONH₂).

2. The agent for prevention or treatment according to Claim 1,
wherein the compound of Formula (I) or a salt thereof is a compound or a salt thereof selected from the group consisting of
pyridin-3-yl 4-{4-[(3-fluorobenzyl)oxy]phenoxy}piperidine-1-carboxylate,
5-{[(4-{4-[(3-fluorobenzyl)oxy]phenoxy}piperidin-1-yl)carbonyl]oxy}nicotinic acid,
5-({[4-(2-phenylethyl)piperidin-1-yl]carbonyl}oxy)nicotinic acid, 5-[({4-[4-(2-cyclohexylethoxy)phenoxy]piperidin-1-yl}carbonyl)oxy]nicotinic acid,
5-[({4-[(E)-2-phenylvinyl]piperidin-1-yl}carbonyl)oxy]nicotinic acid,
5-{[(4-{3-[1-(6-methylpyridin-2-yl)piperidin-4-yl]propyl}piperidin-1-yl)carbonyl]oxy}nicotinic acid,
5-(aminocarbonyl)pyridin-3-yl 4- {2-[3-(aminocarbonyl)phenyl]ethyl}piperidine-1-carboxylate,
5-(aminocarbonyl)pyridin-3-yl 4-(2-{3-[(dimethylamino)carbonyl]phenyl}ethyl)piperidine-1-carboxylate,
5-(aminocarbonyl)pyridin-3-yl 4-{2-[3-(piperidin-1-ylcarbonyl)phenyl]ethyl}piperidine-1-carboxylate,
5-(aminocarbonyl)pyridin-3-yl 4-{2-[3-(pyrrolidin-1-ylcarbonyl)phenyl] ethyl }piperidine-1-carboxylate,
pyridin-3-yl 4-[(2E)-3-phenylprop-2-enoyl]piperazine-1-carboxylate,
pyridin-3-yl 4-(anilinocarbonyl)piperidine-1-carboxylate,
5-(aminocarbonyl)pyridin-3-yl 4-(2-phenylethyl)piperidine-1-carboxylate,
pyridin-3-yl 4-(2-phenylethyl)piperazine-1-carboxylate,
5-(methoxycarbonyl)pyridin-3-yl 4-(2-phenylethyl)piperazine-1-carboxylate,
5-(aminocarbonyl)pyridin-3-yl 4-[2-(3-fluorophenyl)ethyl]piperidine-1-carboxylate,
5-(aminocarbonyl)pyridin-3-yl 4-[2-(3-cyanophenyl)ethyl] piperidine-1-carboxylate,
5-(aminocarbonyl)pyridin-3-yl 4-(5-phenylpentyl)piperazine-1-carboxylate,
pyridin-3-yl 4-(3 -phenyl-1H-1,2,4-triazol-5-yl)piperidine-1-carboxylate,
6-methylpyridin-3-yl 4-[3-(4-fluorophenyl)-1H-1,2,4-triazol-5-yl]piperidine-1-carboxylate,
6-methylpyridin-3-yl 4-[5-(4-fluorophenyl)-1,3-oxazol-2-yl]piperidine-1-carboxylate,
2,6-dimethylpyridin-3-yl 4-[5-(3,4-difluorophenyl)-1,2,4-oxadiazol-3-yl]piperidine-1-carboxylate,
2-methylpyridin-3-yl 4-[3-(2-fluorophenyl)-1H-1,2,4-triazol-5-yl]piperidine-1-carboxylate, and
6-methylpyridin-3-yl 4-(3-phenyl-1H-pyrazol-1-yl)piperidine-1-carboxylate.

3. Use of the compound of Formula (I) or a salt thereof according to Claim 1, for manufacturing an agent for prevention or treatment of interstitial cystitis/bladder pain syndrome and/or chronic nonbacterial prostatitis/chronic pelvic pain syndrome.

4. Use of the compound of Formula (I) or a salt thereof according to Claim 1, for preventing or treating interstitial cystitis/bladder pain syndrome and/or chronic nonbacterial prostatitis/chronic pelvic pain syndrome.

5. The compound of Formula (I) or a salt thereof according to Claim 1, for use in prevention or treatment of interstitial cystitis/bladder pain syndrome and/or chronic nonbacterial prostatitis/chronic pelvic pain syndrome.

6. A method of preventing or treating interstitial cystitis/bladder pain syndrome and/or chronic nonbacterial prostatitis/chronic pelvic pain syndrome, comprising administering an effective amount of the compound of Formula (I) or a salt thereof according to Claim 1 to a mammal.
